# EUROPÄISCHE PATENTSCHRIFT

(11) **EP 0 656 877 B1**
(45) Veröffentlichungstag und Bekanntmachung des Hinweises auf die Patenterteilung: **09.04.1997**
(21) Anmeldenummer: 93919144.1
(22) Anmeldetag: 23.08.1993
(51) Int. Cl.: C07C 41/03, C07C 41/26, C07C 41/42, C07C 41/44, C07C 43/13

(54) **VERFAHREN UND VORRICHTUNG ZUR HERSTELLUNG VON DIGLYCERIN**
PROCESS AND DEVICE FOR PREPARING DIGLYCERINE
PROCEDE ET DISPOSITIF DE PREPARATION DE DIGLYCERINE

(30) Priorität: 25.08.1992 DE 4228147
(43) Veröffentlichungstag der Anmeldung: 14.06.1995
(73) Patentinhaber: Solvay Alkali GmbH, D-30173 Hannover (DE)
(72) Erfinder: JAKOBSON, Gerald, D-47495 Rheinberg (DE); SIEMANOWSKI, Werner, D-47495 Rheinberg (DE)
(74) Vertreter: Lauer, Dieter, Dr.
(86) Internationale Anmeldenummer: EP9302261
(87) Internationale Veröffentlichungsnummer: WO9404478

(56) Entgegenhaltungen:
- EP-A- 0 333 984
- EP-A- 0 374 699
- FR-A- 1 132 490

## Beschreibung

Die vorliegende Erfindung betrifft ein Verfahren zur Herstellung von Diglycerin, das arm an cyclischen Komponenten ist, durch Umsetzung von Glycerin mit Epichlorhydrin bei Temperaturen von
20 bis 120°C, vorzugsweise
50 bis 100°C,
in Gegenwart eines sauren Katalysators und in einem Molverhältnis Glycerin zu Epichlorhydrin von 20 : 1 bis 1 : 1, vorzugsweise 12 : 1 bis 5 : 1, wobei nachfolgend aus dem erhaltenen Reaktionsgemisch das überschüssige Glycerin entfernt wird und das verbleibende Chlorhydrinethergemisch oder Reaktionsgemisch bei einer Temperatur von
60 bis 120°C, vorzugsweise
80 bis 110°C,
(entsprechend dem Gehalt des Reaktionsgemisches an organisch gebundenem Chlor) mit einem alkalisch reagierenden Medium, vorzugsweise einer alkalisch reagierenden wäßrigen Lösung, umgesetzt wird.

Diglycerin (Bis-(2,3-dihydroxy-propyl)-ether) ist ein mehrwertiger Alkohol, der vielseitige Verwendung als Feuchthalte- und Viskositätserhöhungsmittel in Kosmetika und als Ausgangsprodukt für verschiedene Derivate, vor allem grenzflächenaktive Verbindungen, findet. Bei den bekannten Verfahren zur Herstellung von Diglycerin erhält man als Reaktionsprodukt ein Gemisch von Diglycerin mit höheren Polyglycerinen, wobei das Produktgemisch außerdem einen mehr oder weniger hohen Anteil an Glycerin und cyclischen Polyglycerinen aufweist. Für die oben genannten Anwendungen von Diglycerin ist es jedoch erforderlich, daß der Anteil an Diglycerin im Polyglyceringemisch möglichst hoch und die Gehalte an Glycerin und cyclischen Komponenten sehr gering sind.

Aus der EP-A 0 374 699 ist bereits ein Verfahren zur Herstellung von Polyglycerinen, die einen Anteil von mehr als 50 Gew.-% Diglycerin aufweisen und arm an cyclischen Komponenten sind, bekannt. Die Herstellung erfolgt durch Umsetzung von Glycerin mit Epichlorhydrin bei Temperaturen von 20 bis 140°C, vorzugsweise 60 bis 100°C, in Gegenwart eines sauren Katalysators und in einem Molverhältnis von Glycerin zu Epichlorhydrin von 10 : 1 bis 1 : 1, vorzugsweise 6 : 1 bis 1,4 : 1, wobei nachfolgend das erhaltene, gegebenenfalls von überschüssigem Glycerin befreite Reaktionsgemisch bei einer Temperatur von 50 bis 120°C, vorzugsweise 60 bis 95°C, entsprechend dem Gehalt an organisch gebundenem Chlor, mit einem alkalisch reagierenden Medium, vorzugsweise einer alkalisch reagierenden wäßrigen Lösung, umgesetzt wird. Anschließend erfolgt eine weitere Aufarbeitung oder Reinigung des erhaltenen Reaktionsgemisches, wobei nach Wasserzugabe das Glycerin-Polyglyceringemisch über einen oder mehrere Ionenaustauscher entsalzt, durch Destillation entwässert und schließlich durch fraktionierte Destillation in Glycerin, Diglycerin und höhere Polyglycerine aufgetrennt wird.

Aufgabe der vorliegenden Erfindung war es, das bekannte Verfahren in der Weise zu modifizieren, daß bei kontinuierlicher Reaktionsführung eine weitere Erhöhung des Diglyglyceringehaltes in dem Polyglyceringemisch erreicht wird, so daß ein Produkt erhalten wird, das vorzugsweise einen Anteil von mehr als 80 Gew.-% Diglycerin aufweist und das arm an cyclischen Komponenten ist. Darüber hinaus sollte die Steigerung des Diglyceringehaltes in einem wirtschaftlichen Verfahren realisiert werden, das zugleich eine vereinfachte Aufarbeitung des Reaktionsgemisches und eine vorteilhafte Reduzierung von unerwünschten Bestandteilen erlaubt, so daß die Qualität des Reaktionsproduktes ebenfalls verbessert werden kann.

Erfindungsgemäß wurde festgestellt, daß dieser Aufgabe ein Verfahren gerecht wird, bei dem die Herstellung von Diglycerin, das arm an cyclischen Komponenten ist, durch Umsetzung von Glycerin mit Epichlorhydrin bei Temperaturen von
20 bis 120°C, vorzugsweise
50 bis 100°C,
in Gegenwart eines sauren Katalysators und in einem Molverhältnis Glycerin zu Epichlorhydrin von 20 : 1 bis 1 : 1, vorzugsweise 12 : 1 bis 5 : 1, erfolgt, wobei in einer kontinuierlichen Reaktionsführung das zur Umsetzung erforderliche Epichlorhydrin an mehreren, räumlich in Durchflußrichtung voneinander getrennten Stellen oder Reaktionszonen in mindestens einen Durchlaufreaktor eingeleitet wird. Anschließend wird das umgesetze Reaktionsgemisch in mindestens einer Verdampferanlage von überschüssigem Glycerin weitgehend befreit oder getrennt und das verbleibende Chlorhydrinethergemisch bei einer Temperatur von
60 bis 120°C, vorzugsweise
80 bis 110°C,
in einer ersten Stufe mit einer wäßrigen Alkalicarbonatlösung in einem Überschuß von
5 bis 40 Mol-%, vorzugsweise
15 bis 25 Mol-%,
berechnet auf das organisch gebundene Chlor der Chlorhydrinnether, und in einem pH-Bereich von
6,5 bis 9,5, vorzugsweise
7 bis 8,5,
hydrolysiert und in einer zweiten Stufe mit Alkalihydroxid, vorzugsweise einer wäßrigen Alkalihydroxidlösung, bei einem pH-Wert über 10 die Hydrolyse zu Ende geführt. Nach Abschluß oder weitgehendem Abschluß der Hydrolyse wird die Rohproduktlösung durch Zugabe einer Mineralsäure, vorzugsweise Salzsäure, auf einen pH-Wert von
5,5 bis 8, vorzugsweise
6 bis 7,
eingestellt und die erhaltene Lösung eingedampft, wobei man die ausfallenden Salze abtrennt und nachfolgend die unter 8 Gew.-% Restgehalt anorganische Salze enthaltende Diglycerinlösung in mindestens einem Kurzwegverdampfer von cyclischem Diglycerin und restlichen Anteilen an Glycerin destillativ befreit.

Das erfindungsgemäße Verfahren ermöglicht es, die Umsetzung von Epichlorhydrin mit Glycerin kontinuierlich durchzuführen und ein Diglycerin herzustellen, das nicht nur arm an cyclischen Komponenten, sondern das schon als Rohprodukt vor der letztendlichen destillativen Behandlung einen sehr geringen Gehalt an Glycerin aufweist, wobei der Anteil an offenkettigem Diglycerin bereits in dem Rohprodukt über 80 Gew.-% beträgt.

Die vorteilhafte Ausbeuteverbesserung an Diglycerin wird insbesondere durch zwei erfindungsgemäße Maßnahmen erreicht: zum einen leitet man das zur Umsetzung erforderliche Epichlorhydrin an mehreren, räumlich in Durchflußrichtung voneinander getrennten Stellen oder Reaktionszonen in mindestens einen Durchlaufreaktor ein, so daß der Epichlorhydrinstrom in mehrere Teilströme aufgeteilt wird, wodurch in dem Durchlaufreaktor, entsprechend der Zahl der eingeleiteten Teilströme, mehrere Reaktionszonen oder -zentren gebildet werden, an denen die Umsetzung von Glycerin und Epichlorhydrin zu Diglycerin als Hauptprodukt der Synthese erfolgt; zum anderen wird das nicht umgesetzte Glycerin sofort nach Austritt des Reaktionsgemisches aus dem Durchlaufreaktor in einer Desillationsanlage abgetrennt. Die weitgehende Entfernung des Glycerins mit verhältnismäßig einfachen destillativen Methoden ist nur in dieser Phase des erfindungsgemäßen Verfahrens möglich. Zudem kann man auf diese Weise den Volumenstrom, der in einer nachfolgenden Hydrolyse mit alkalischen Mitteln behandelt wird, wesentlich reduzieren.

Nach einer vorteilhaften Ausführungsform wird der Gehalt an überschüssig eingesetztem Glycerin in dem Reaktionsgemisch in mindestens einer Verdampferanlage, vorzugsweise einer kontinuierlich betriebenen Verdampferanlage, auf unter 20 Gew.-% (bezogen auf das Reaktionsgemisch), vorzugsweise unter 10 Gew.-%, und insbesondere auf unter 3 Gew.-% gesenkt.

Als Verdampferanlagen eignen sich insbesondere Dünnschichtverdampfer und/oder Kurzwegverdampfer, vorzugsweise eine Kombination aus diesen beiden Verdampferarten. Das als Kopfprodukt anfallende Glycerin hat einen Massengehalt von >98 % und kann in den Prozeß zurückgeführt werden.

Die an den vorstehenden Verfahrensschritt anschließende, erfindungsgemäß zweistufige Hydrolyse des Chlorhydrinethergemisches erlaubt eine vollständige Substitution des organisch gebundenen Chlors durch jeweils eine OH-Gruppe, so daß die entsprechenden Polyglycerinverbindungen erhalten werden.

Bei den bereits bekannten Verfahren hatte sich gezeigt, daß in einer einzigen Hydrolysestufe mit einer Alkalicarbonatlösung keine vollständige Umsetzung der Chlorhydrinether erreicht werden kann, so daß hierbei bis zu 10 % der Chlorhydrinether in der Reaktionslösung verbleiben und ein Diglycerin erhalten wird, das noch organisch gebundenes Chlor enthält. Eine destillative Trennung der Chlorhydrinether vom Diglycerin ist mit einem Dünnschicht- oder Kurzwegverdampfer nicht möglich.

Die vorteilhafte vollständige Umsetzung des organisch gebundenen Chlors erfolgt erfindungsgemäß insbesondere in der zweiten Hydrolysestufe durch eine Nachbehandlung der restlichen Chlorhydrinether mit Alkalihydroxid bei einem pH-Wert über 10. Erfindungsgemäß wurde dabei festgestellt, daß für die Wirksamkeit der zweiten Behandlungsstufe die Einstellung des pH-Wertes >10 entscheidend ist.

Um im Anschluß an die Hydrolyse eine weitgehende Entsalzung des Produktgemisches und somit eine Erniedrigung der Viskosität der Rohproduktlösung nach der Eindampfung zu erreichen, wird die alkalische Rohproduktlösung mit einer Mineralsäure, vorzugsweise Salzsäure, auf einen pH-Bereich von 6,5 bis 9,5, vorzugsweise 7 bis 8,5 eingestellt. Auf diese Weise erhält man nach Eindampfung der Lösung gut filtrierbare Salze.

Nach einer bevorzugten Ausführungsform des erfindungsgemäßen Verfahrens wird die mit Mineralsäure behandelte Rohproduktlösung in einem Kurzwegverdampfer eingedampft und anschließend von ausfallenden Salzen durch Filtration oder Zentrifugation befreit.

Die erhaltene Roh-Diglycerinlösung wird durch diesen Verfahrensschritt so weit entsalzt, daß durch eine anschließende, einfache Destillation in mindestens einem Kurzwegverdampfer cyclisches Diglycerin und restliche Anteile an Glycerin entfernt werden können und schließlich Diglycerin in hoher Ausbeute und Reinheit erhalten wird.

Der Anteil an Diglycerin in dem Rohprodukt kann nach einer weiteren vorteilhaften Ausführungsform des erfindungsmäßen Verfahrens dadudrch gesteigert werden, daß das Epichlorhydrin in zwei oder mehr Teilströmen in mindestens einen, durch Querschnittsverengungen in zwei oder mehr Einzelkammern oder Reaktionszonen unterteilten Durchlaufreaktor eingeleitet wird, wobei vorzugsweise in jede Einzelkammer oder Reaktionszone mindestens ein Teilstrom eingeführt wird.

Die erfindungsgemäße Erhöhung des Diglyceringehaltes in dem Rohprodukt kann auch dadurch gesteigert werden, daß der Gesamtstrom des zur Umsetzung erforderlichen Epichlorhydrins in zwei Teilströme aufgeteilt wird und ein erster Teilstrom an mehreren, räumlich in Durchflußrichtung voneinander getrennten Stellen oder Reaktionszonen in einen ersten Durchlaufreaktor eingeleitet und anschließend das Reaktionsgemisch kontinuierlich in einen zweiten Durchlaufreaktor überführt wird, wo der zweite Teilstrom des Epichlorhydrins, vorzugsweise an mehreren, räumlich in Durch-flußrichtung voneinander getrennten Steilen oder Reaktionszonen, zudosiert wird.

In der ersten Hydrolysestufe des erfindungsgemäßen Verfahrens wird nach einer vorteilhaften Ausführungsform eine mehr als 1,5-molare, vorzugsweise mehr als 2-molare, Sodalösung eingesetzt, um bereits in diesem ersten Hydrolyseschritt eine hohe Umsetzungsrate des organisch gebundenen Chlors zu erreichen.

In der zweiten Hydrolysestufe wird vorzugsweise eine mehr als 10 gew.%ige Natronlauge; insbesondere eine mehr als 35 gew.-%ige Natronlauge eingesetzt.

Nach einer bevorzugten Ausführungsform des erfindungsgemäßen Verfahrens wird die Verweilzeit des Reaktionsgemisches in der ersten Stufe der Hydrolyse auf eine Zeit von mehr als 1 Stunde, vorzugsweise mehr als 1,5 Stunden, und die Verweilzeit in der zweiten Hydrolysestufe auf mehr als 0,5 Stunden, vorzugsweise mehr als 0,75 Stunden, eingestellt.

Zur vollständigen Entsalzung kann die destillativ von Glycerin und cyclischem Diglycerin befreite Produktlösung mit Ionenaustauschern, vorzugsweise einer Kombination aus mehreren basischen Kationenaustauschern und mindestens einem sauren Anionenaustauscher, behandelt werden, wobei die Diglycerin/Polyglycerinlösung zuvor mit Wasser verdünnt wird.

Nach Durchleitung der Produktlösung durch die Ionenaustauscher kann wiederum eine Entwässerung durch Eindampfen erfolgen und anschließend eine destillative Auftrennung in Diglycerin und höhere Polyglycerine durchgeführt werden.

Die Erfindung betrifft weiterhin eine verbesserte Vorrichtung zur Durchführung des erfindungsgemäßen Verfahrens. Diese Vorrichtung enthält oder umfaßt
einen Reaktor oder Reaktionsbehälter, vorzugsweise einen vertikal angeordneten Durchlaufreaktor mit zylindrischem Reaktorgehäuse, mit mindestens einem Einlaß für das Glycerin und den Katalysator am unteren Ende und mit mindestens einem Auslaß für das Reaktionsgemisch am oberen Ende, mit mindestens einer in dem Reaktor oder Reaktionsbehälter koaxial und zentral angeordneten Rührwelle, auf der mindestens eine Rühreinrichtung befestigt ist, und mit einer Zuführleitung für das Epichlorhydrin, die parallel zur Rührwelle angeordnet ist und in mehreren Leitungsenden oder -öffnungen auf verschiedenen Niveauhöhen in den Reaktorraum mündet,
mindestens eine Verdampferanlage, vorzugsweise einen Dünnschicht- und/oder Kurzwegverdampfer,
mindestens einen Reaktionsbehälter zur Durchführung der Hydrolyse,
mindestens einen Reaktionsbehälter zur Durchführung der Nachhydrolyse und
mindestens einen weiteren Kurzwegverdampfer,
wobei der Reaktionsraum durch Querschnittsverengungen in mindestens zwei, vorzugsweise mehr als drei, Reaktionszonen oder -kammern unterteilt ist, die Zuführleitung für das Epichlorhydrin innerhalb des Reaktorraumes, in einem Abstand von dem äußeren Umfang der Rühreinrichtung oder -einrichtungen, angeordnet ist und in jeder Reaktionszone oder -kammer mindestens ein Leitungsende oder eine Leitungsöffnung der Zuführleitung für das Epichlorhydrin mündet und mindestens eine Rühreinrichtung an der Rührwelle befestigt ist.

Die erfindungsgemäße Vorrichtung und ihre vorteilhaften Ausführungsformen werden im Folgenden anhand der Zeichnungsfigur 1 beschrieben.

Der Reaktor oder Reaktionsbehälter **(1)**, der vorzugsweise als vertikal angeordneter Durchlaufreaktor, oder auch als Reaktionskolonne, mit zylindrischem Reaktorgehäuse ausgebildet ist, weist an seinem unteren Ende, vorzugsgweise im Reaktorboden, einen Einlaß oder eine Zuführleitung auf, durch die das Reaktionsedukt Glycerin **(9)** mit dem zugesetzten Katalysator in den Reaktor eingetragen wird; am oberen Ende des Reaktors, vorzugsweise oberhalb der obersten oder höchstliegenden Querschnittsverengung, befindet sich ein Auslaß oder eine Abführleitung, durch die das gebildete Reaktionsgemisch **(3)** aus dem Reaktor ausgetragen wird. Die Einleitung der Eduktströme und/oder die Ableitung des Produktstromes kann durch den Einsatz von Pumpen unterstützt werden.

Weiterhin ist zentral in dem Reaktorraum eine koaxiale Rührwelle **(2)** angeordnet, die kontinuierlich drehbar und auf der mindestens eine Rühreinrichtung **(10)** befestigt ist. Eine solche Rühreinrichtung kann beispielsweise aus einem Scheibenrührer bestehen. Achsenparallel zur Rührwelle weist die erfindungsgemäße Vorrichtung eine rohrförmige Leitung **(7)** auf, durch die das Epichlorhydrin **(6)** in den Reaktorraum geführt wird. Erfindungsgemäß ist die Zuführleitung **(7)** innerhalb des Reaktorraumes, in einem Abstand zum äußeren Umfang der Rühreinrichtung oder -einrichtungen, angeordnet. Sie mündet in mehreren Leitungsenden oder -öffnungen **(8)** auf verschiedenen Niveauhöhen in den Reaktorraum, wobei letzterer durch Querschnittsverengungen in mindestens zwei, vorzugsweise mehr als drei, Reaktionszonen oder -kammern **(4)** unterteilt ist, und in jeder Reaktionszone oder -kammer mindestens ein Leitungsende oder eine Leitungsöffnung **(8)** der Zuführleitung für das Epichlorhydrin und mindestens eine auf der Rührwelle befestigte Rühreinrichtung **(10)** vorgesehen sind.

Durch die Aufteilung des Reaktionsraumes in mehrere Reaktionszonen, in die jeweils mindestens ein Teilstrom des Epichlorhydrins durch jeweils ein Leitungsende oder eine Leitungsöffnung der Zuführleitung **(7)** in das Gemisch aus Glycerin und Katalysator eingetragen wird, bilden sich, entsprechend der Zahl der eingeleiteten Teilströme, mehrere Reaktionszentren, in denen die Umsetzung von Glycerin und Epichlorhydrin mit einer überraschend höheren Ausbeute an Diglycerin als bei den bekannten Verfahren erfolgt.

Diese höhere Ausbeute an Diglycerin erhält man erfindungsgemäß zwar schon dadurch, daß man das Epichlorhydrin an mehreren, räumlich in Durchflußrichtung voneinander getrennten Stellen in den Durchlaufreaktor einleitet, doch wird durch die Bildung einzelner Reaktionszonen oder -kammern durch die Einführung von Querschnittsverengungen und durch die kontinuierliche Durchmischung des Reaktionsgemisches in jeder Kammer mit Hilfe mindestens einer Rühreinrichtung ein sehr günstiges Verhältnis von Glycerin zu Epichlorhydrin eingestellt, so daß die Ausbeute durch die erfindungsgemäße Ausgestaltung des Durchlaufreaktors nochmals verbessert werden konnte.

Diese Ausbeuteverbesserung wird weiterhin dadurch unterstützt, daß nach einer bevorzugten Ausführungsform der erfindungsgemäßen Vorrichtung die Querschnittsverengungen in besonderer Weise ausgebildet sind. Sie bestehen danach aus Zwischenbodenplatten, Umlenkblechen oder Trennscheiben **(5)**, die mehrere Durchbrüche oder Durchtrittsöffnungen für das Reaktionsgemisch aufweisen. Hierdurch wird eine ungehinderte Durchmischung der in den einzelnen Reaktionszentren gebildeten Produktlösungen und ein frei fließender, aufwärtsgerichteter Strom des Reaktionsgemisches im Reaktorraum gewährleistet.

Nach einer weiteren vorteilhaften Ausführungsform der erfindungsgemäßen Vorrichtung sind die Zwischenbodenplatten, Umlenkbleche oder Trennscheiben kreisförmig ausgebildet und waagerecht auf verschiedenen Niveauhöhen in dem Reaktorraum angeordnet, wobei die Zwischenbodenplatten, Umlenkbleche oder Trennscheiben jeweils eine zentrale Öffnung für die Durchführung der Rührwelle und eine Öffnung für die Durchführung der Zuleitung für das Epichlorhydrin aufweisen.

Die freie Führung und Durchmischung der Produktströme und/oder der Reaktionskomponenten kann weiterhin dadurch verbessert werden, daß zwischen dem Rand der Zwischenbodenplatten, Umlenkbleche oder Trennscheiben und der Reaktorwand ein nicht abgedichteter Spalt von ausgebildet ist.

Des weiteren hat es sich als vorteilhaft erwiesen, wenn die Zwischenbodenplatten, Umlenkbleche oder Trennscheiben in etwa den gleichen Abstand voneinander aufweisen, so daß etwa gleich große Reaktionszonen oder -kammern entstehen.

Gemäß einer weiteren bevorzugten Ausführungsform der erfindungsgemäßen Vorrichtung wird die Durchmischung der Reaktionskomponenten dadurch gesteigert, daß die Leitungsenden oder -öffnungen der Zuführleitung für das Epichlorhydrin als Venturidüsen ausgebildet sind.

In der erfindungsgemäßen Vorrichtung kann ein Einzelreaktor bzw. eine einzelne Reaktionskolonne eingesetzt werden. Es können aber auch zwei oder mehr Reaktoren der erfindungsgemäßen Art hintereinandergeschaltet verwendet werden, womit nochmals eine Auftrennung des Epichlorhydringesamtsstroms in eine höhere Zahl von Teilströmen vorgenommen wird.

### Erläuterungen zur Figur

Figur 1:
Schematische Darstellung eines Reaktionsbehälters zur Erläuterung der erfindungsgemäßen Vorrichtung und des erfindungsgemäßen Verfahrens; die Bedeutung der Bezugszeichen (1) bis (10) ergibt sich aus der vorstehenden Beschreibung

Das nachfolgende Ausführungsbeispiel soll die Erfindung erläutern, ohne sie darauf zu beschränken.

### Ausführungsbeispiel

In einen Durchlaufreaktor (1-1-Doppelwandreaktor aus Glas mit Rührer und zwei räumlich in Durchflußrichtung voneinander getrennten Zudosierungsleitungen für das Epichlorhydrin) werden innerhalb von einer Stunde 5,526 kg (≙ 60 mol), mit einer katalytischen Menge an Schwefelsäure versetztem Glycerin eingeleitet und 0,694 kg (≙ 7,5 mol) Epichlorhydrin in zwei Teilströmen in den Durchflußreaktor zudosiert, wobei durch Kühlung über den Doppelmantel eine Reaktionstemperatur von 60° bis 70°C eingestellt wird.

Der Ablauf des Reaktors wird einer Kombination aus Dünnschicht- und Kurzwegverdampfer zugeleitet und das überschüssig eingesetzte Glycerin destillativ aus dem Reaktionsgemisch (Restgehalt an Glycerin im Chlorhydrinethergemisch < 3%) entfernt. Der Sumpfablauf der Verdampferanlage wird in einem Pufferbehälter geleitet und anschließend das Chlorhydrinethergemisch portionsweise in einer 2-stufigen Hydrolyse,
- - 1. Stufe:: Zugabe von 2,5-molarer Sodalösung (20 Mol-% Überschuß an Natriumcarbonat, Reaktionszeit ca. 2 h, Reaktionstemperatur ca. 95°C, pH-Wert 7-8,5)
- - 2. Stufe:: Zugabe von 50 %iger Natronlauge (ca. 10 bis 15 %, bezogen auf die eingesetzte Menge an 2,5-molarer Sodalösung, Reaktionszeit ca. 1 h, pH-Wert 10,5),
zu rohem Diglycerin/Polyglyceringemisch überführt.

Die erhaltene wäßrige Diglycerin/Polyglycerinlösung wird mit konzentrierter Salzsäure auf einen pH-Wert von 6 bis 7 eingestellt und anschließend im Vakuum eingedampft, wobei ausfallende Anteile an Salz durch Filtration abgetrennt werden.

Die noch salzhaltige Produktlösung (Restsalzgehalt unter 8 Gew.-%) wird in einem Dünnschichtverdampfer destillativ von Glycerin und cyclischen Diglycerin befreit und der Sumpfablauf des Verdampfers über eine Kombination von sauren und basischen Ionenaustauschern (nach vorheriger Zugabe von Wasser) vollständig entsalzt, anschließend eingedampft und destillativ in Diglycerin und höhere Polyglycerine aufgetrennt.

Eine GC-Analyse der entsalzten und entwässerten Roh-Diglycerin/Polyglycerinlösung nach der zweistufigen Hydrolyse ergibt folgende Gehalte (Zahlenwerte jeweils bezogen auf g/kg):

| | |
|---|---|
| Glycerin | 20 |
| cycl. Diglycerin | 30 |
| Diglycerin | 830 |
| cycl. Triglycerin | 20 |
| Triglycerin | 80 |
| cycl. Tetraglycerin | 5 |
| Tetraglycerin | 10 |
| Pentaglycerin | 5 |

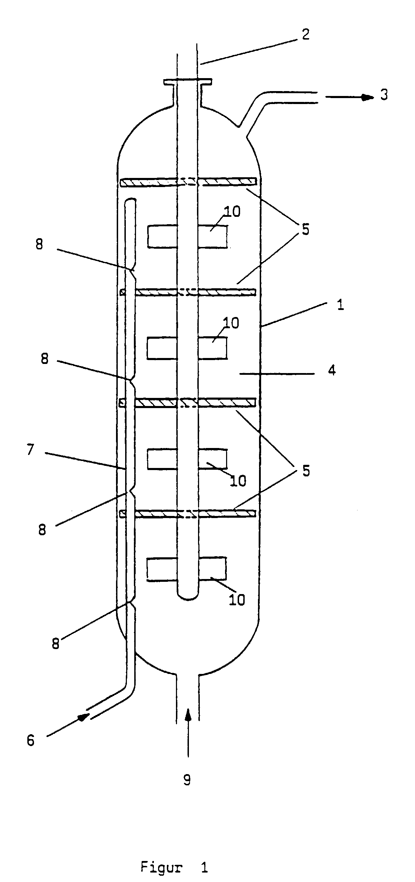

## Patentansprüche

1. Verfahren zur Herstellung von Diglycerin, das arm an cyclischen Komponenten ist, durch Umsetzung von Glycerin mit Epichlorhydrin bei Temperaturen von
20 bis 120°C, vorzugsweise
50 bis 100°C,
in Gegenwart eines sauren Katalysators und in einem Molverhältnis Glycerin zu Epichlorhydrin von 20 : 1 bis 1 : 1, vorzugsweise 12 : 1 bis 5 : 1, wobei nachfolgend aus dem erhaltenen Reaktionsgemisch das überschüssige Glycerin entfernt wird und das verbleibende Chlorhydrinethergemisch oder Reaktionsgemisch bei einer Temperatur von
60 bis 120°C, vorzugsweise
80 bis 110°C,
(entsprechend dem Gehalt des Reaktionsgemisches an organisch gebundenem Chlor) mit einem alkalisch reagierenden Medium, vorzugsweise einer alkalisch reagierenden wäßrigen Lösung, umgesetzt wird, dadurch gekennzeichnet, daß zur kontinuierlichen Herstellung von Diglycerin das zur Umsetzung erforderliche Epichlorhydrin an mehreren, räumlich in Durchflußrichtung voneinander getrennten Stellen oder Reaktionszonen in mindestens einen Durchlaufreaktor eingeleitet wird, daß das umgesetzte Reaktionsgemisch in mindestens einer Verdampferanlage von überschüssigem Glycerin weitgehend befreit oder getrennt und das verbleibende Chlorhydrinethergemisch in mindestens einer ersten Stufe mit einer Alkalicarbonatlösung in einem Überschuß von
5 bis 40 Mol-%, vorzugsweise
15 bis 25 Mol-%,
berechnet auf das organisch gebundene Chlor der Chlorhydrinether, und in einem pH-Bereich von
6,5 bis 9,5, vorzugsweise
7 bis 8,5,
hydrolysiert und in einer zweiten Stufe mit Alkalihydroxid, vorzugsweise einer wäßrigen Alkalihydroxidlösung, bei einem pH-Wert über 10 die Hydrolyse zu Ende geführt wird, daß nach Abschluß oder weitgehendem Abschluß der Hydrolyse durch Zugabe einer Mineralsäure, vorzugsweise Salzsäure, die Rohproduktlösung auf einen pH-Wert von
5,5 bis 8, vorzugsweise
6 bis 7,
eingestellt und die erhaltene Lösung eingedampft wird, wobei man ausfallende Salze abtrennt und nachfolgend die unter 8 Gew.-% Restgehalt anorganische Salze enthaltende Diglycerinlösung in mindestens einem Kurzwegverdampfer von cyclischem Diglycerin und restlichen Anteilen an Glycerin destillativ befreit.

2. Verfahren nach Anspruch 1, dadurch gekennzeichnet, daß das Epichlorhydrin in zwei oder mehr Teilströmen in mindestens einen, durch Querschnittsverengungen in zwei oder mehr Einzelkammern oder Reaktionszonen unterteilten Durchlaufreaktor eingeleitet wird, wobei vorzugsweise in jede Einzelkammer oder Reaktionszone mindestens ein Teilstrom eingeführt wird.

3. Verfahren nach den Ansprüchen 1 oder 2, dadurch gekennzeichnet, daß der Gesamtstrom des zur Umsetzung erforderlichen Epichlorhydrins in zwei Teilströme aufgeteilt wird und ein erster Teilstrom an mehreren, räumlich in Durchflußrichtung voneinander getrennten Stellen oder Reaktionszonen in einen ersten Durchlaufreaktor eingeleitet und anschließend das Reaktionsgemisch kontinuierlich in einen zweiten Durchlaufreaktor überführt wird, wo der zweite Teilstrom des Epichlorhydrins, vorzugsweise an mehreren, räumlich in Durchflußrichtung voneinander getrennten Stellen oder Reaktionszonen, zudosiert wird.

4. Verfahren nach einem oder mehreren der Ansprüche 1 bis 3, dadurch gekennzeichnet, daß der Gehalt (in dem Reaktionsgemisch nach der Umsetzung von Glycerin mit Epichlorhydrin) an überschüssig eingesetztem Glycerin in mindestens einer Verdampferanlage, vorzugsweise einer kontinuierlich betriebenen Verdampferanlage, auf unter 20 Gew.-% (bezogen auf das Reaktionsgemisch), vorzugsweise unter 10 Gew.-%, gesenkt wird.

5. Verfahren nach Anspruch 4, dadurch gekennzeichnet, daß als Verdampferanlage ein Dünnschicht- und/oder ein Kurzwegverdampfer, vorzugsweise eine Kombination aus beiden Verdampferarten, eingesetzt und das als Kopfprodukt anfallende Glycerin in den Prozeß zurückgeführt wird.

6. Verfahren nach einem oder mehreren der Ansprüche 1 bis 5, dadurch gekennzeichnet, daß als Alkalicarbonatlösung eine
mehr als 1,5 molare, vorzugsweise
mehr als 2 molare,
Sodalösung verwendet wird.

7. Verfahren nach einem oder mehreren der Ansprüche 1 bis 6, dadurch gekennzeichnet, daß als wäßrige Alkalihydroxidlösung eine mehr als 10 gew.-%ige Natronlauge, vorzugsweise eine mehr als 35 gew.-%ige Natronlauge, verwendet wird.

8. Verfahren nach einem oder mehreren der Ansprüche 1 bis 7, dadurch gekennzeichnet, daß die Verweilzeit des Reaktionsgemisches in der ersten Stufe der Hydrolyse auf eine Zeit von mehr als 1 Stunde, vorzugsweise mehr als 1,5 Stunden, und die Verweilzeit in der zweiten Hydrolysestufe auf mehr als 0,5 Stunden, vorzugsweise mehr als 0,75 Stunden, eingestellt wird.

9. Verfahren nach einem oder mehreren der Ansprüche 1 bis 8, dadurch gekennzeichnet, daß die mit einer Mineralsäure behandelte Rohproduktlösung in einem Kurzwegverdampfer eingedampft und anschließend von den ausfallenden Salzen durch Filtration oder Zentrifugation befreit wird.

10. Vorrichtung zur Durchführung des Verfahrens nach den Ansprüchen 1 bis 9, enthaltend
einen Reaktor oder Reaktionsbehälter, vorzugsweise einen vertikal angeordneten Durchlaufreaktor mit zylindrischem Reaktorgehäuse, mit mindestens einem Einlaß für das Glycerin und den Katalysator am unteren Ende und mit mindestens einem Auslaß für das Reaktionsgemisch am oberen Ende, mit mindestens einer in dem Reaktor oder Reaktionsbehälter koaxial und zentral angeordneten Rührwelle, auf der mindestens eine Rühreinrichtung befestigt ist, und mit einer Zuführleitung für das Epichlorhydrin, die parallel zur Rührwelle angeordnet ist und in mehreren Leitungsenden oder -öffnungen auf verschiedenen Niveauhöhen in den Reaktorraum mündet,
mindestens eine Verdampferanlage, vorzugsweise einen Dünnschicht- und/oder Kurzwegverdampfer,
mindestens einen Reaktionsbehälter zur Durchführung der Hydrolyse,
mindestens einen Reaktionsbehälter zur Durchführung der Nachhydrolyse und
mindestens einen weiteren Kurzwegverdampfer, dadurch gekennzeichnet, daß der Reaktionsraum, in dem die Umsetzung von Epichlorhydrin mit Glycerin durchgeführt wird, durch Querschnittsverengungen in mindestens zwei, vorzugsweise mehr als drei, Reaktionszonen oder -kammern unterteilt ist, daß die Zuführleitung für das Epichlorhydrin innerhalb des Reaktorraumes, in einem Abstand von dem äußeren Umfang der Rühreinrichtung oder -einrichtungen, angeordnet ist und daß in jeder Reaktionszone oder -kammer mindestens ein Leitungsende oder eine Leitungsöffnung der Zuführleitung für das Epichlorhydrin mündet und mindestens eine Rühreinrichtung an der Rührwelle befestigt ist.

11. Vorrichtung nach Anspruch 10, dadurch gekennzeichnet, daß die Querschnittsverengungen aus Zwischenbodenplatten, Umlenkblechen oder Trennscheiben, die mehrere Durchbrüche oder Durchtrittsöffnungen für das Reaktionsgemisch aufweisen, bestehen.

12. Vorrichtung nach den Ansprüchen 10 oder 11, dadurch gekennzeichnet, daß die Zwischenbodenplatten, Umlenkbleche oder Trennscheiben kreisförmig ausgebildet sind und waagerecht auf verschiedenen Niveauhöhen in dem Reaktorraum angeordnet sind, wobei die Zwischenbodenplatten, Umlenkbleche oder Trennscheiben jeweils eine zentrale öffnung für die Durchführung der Rührwelle und eine Öffnung für die Durchführung der Zuleitung für das Epichlorhydrin aufweisen.

13. Vorrichtung nach einem oder mehreren der Ansprüche 10 bis 12, dadurch gekennzeichnet, daß zwischen dem Rand der Zwischenbodenplatten, Umlenkbleche oder Trennscheiben und der Reaktorwand ein nicht abgedichteter Spalt ausgebildet ist.

14. Vorrichtung nach einem oder mehreren der Ansprüche 10 bis 13, dadurch gekennzeichnet, daß die Zwischenbodenplatten, Umlenkbleche oder Trennscheiben in etwa den gleichen Abstand voneinander aufweisen.

15. Vorrichtung nach einem oder mehreren der Ansprüche 10 bis 14, dadurch gekennzeichnet, daß die Leitungsenden oder -öffnungen der Zuführleitung für das Epichlorhydrin als Venturidüsen ausgebildet sind.

## Claims

1. A method for preparing diglycerol which is low in cyclic constituents, by reacting glycerol with epichlorohydrin at temperatures of
20 to 120°C, preferably
50 to 100°C,
in the presence of an acidic catalyst and in a molar ratio of glycerol to epichlorohydrin of 20 : 1 to 1 : 1, preferably 12 : 1 to 5 : 1, wherein subsequently the excess glycerol is removed from the resulting reaction mixture and the remaining chlorohydrin ether mixture or reaction mixture is reacted with an alkaline-reacting medium, preferably an alkaline-reacting aqueous solution, at a temperature of
60°C to 120°C, preferably
80 to 110°C,
(corresponding to the content of organically bound chlorine in the reaction mixture), characterised in that for the continuous production of diglycerol the epichlorohydrin required for reaction is introduced into at least one continuous reactor at a plurality of points or reaction zones separated spatially from each other in the direction of throughflow, that the reacted reaction mixture is largely freed of or separated from excess glycerol in at least one evaporator unit and the remaining chlorohydrin ether mixture is hydrolysed in at least one first stage with an alkali carbonate solution in an excess of
5 to 40 mole %, preferably
15 to 25 mole %,
calculated relative to the organically bound chlorine of the chlorohydrin ether, and in a pH range of
6.5 to 9.5, preferably
7 to 8.5,
and the hydrolysis is completed in a second stage with alkali hydroxide, preferably an aqueous alkali hydroxide solution, at a pH value of over 10, that once the hydrolysis has ended or largely ended the crude product solution is set to a pH value of
5.5 to 8, preferably
6 to 7,
by addition of a mineral acid, preferably hydrochloric acid, and the resulting solution is evaporated, with salts which are produced being separated off and subsequently the diglycerol solution containing less than 8% by weight residual content of inorganic salts is freed of cyclic diglycerol and residual contents of glycerol by distillation in at least one short-path evaporator.

2. A method according to Claim 1, characterised in that the epichlorohydrin is introduced in two or more partial streams into at least one continuous reactor subdivided by constrictions in cross-section into two or more individual chambers or reaction zones, with preferably at least one partial stream being introduced into each individual chamber or reaction zone.

3. A method according to Claims 1 or 2, characterised in that the complete stream of the epichlorohydrin required for reaction is divided up into two partial streams and a first partial stream is introduced into a first continuous reactor at a plurality of points or reaction zones separated spatially from each other in the direction of throughflow and then the reaction mixture is transferred continuously into a second continuous reactor, where the second partial stream of the epichlorohydrin is metered in preferably at a plurality of points or reaction zones separated spatially from each other in the direction of throughflow.

4. A method according to one or more of Claims 1 to 3, characterised in that the content (in the reaction mixture after the reaction of glycerol with epichlorohydrin) of glycerol used in excess is reduced in at least one evaporator unit, preferably a continuously operated evaporator unit, to less than 20% by weight (relative to the reaction mixture), preferably less than 10% by weight.

5. A method according to Claim 4, characterised in that a film evaporator and/or a short-path evaporator, preferably a combination of both types of evaporator, is used as the evaporator unit and the glycerol produced as overhead product is recycled into the process.

6. A method according to one or more of Claims 1 to 5, characterised in that a
more than 1.5 molar, preferably
more than 2 molar,
soda solution is used as alkali carbonate solution.

7. A method according to one or more of Claims 1 to 6, characterised in that a more than 10% by weight sodium hydroxide solution, preferably a more than 35% by weight sodium hydroxide solution, is used as aqueous alkali hydroxide solution.

8. A method according to one or more of Claims 1 to 7, characterised in that the dwell time of the reaction mixture in the first stage of the hydrolysis is set to a time of more than 1 hour, preferably more than 1.5 hours, and the dwell time in the second hydrolysis stage is set to more than 0.5 hours, preferably more than 0.75 hours.

9. A method according to one or more of Claims 1 to 8, characterised in that the crude product solution treated with a mineral acid is evaporated in a short-path evaporator and is then freed of the resulting salts by filtration or centrifuging.

10. An apparatus for performing the method according to Claims 1 to 9, comprising
a reactor or reaction vessel, preferably a vertically arranged continuous reactor with cylindrical reactor housing, with at least one inlet for the glycerol and the catalyst at the lower end and with at least one outlet for the reaction mixture at the upper end, with at least one stirrer shaft arranged coaxially and centrally in the reactor or reaction vessel, to which shaft at least one stirrer mechanism is attached, and with a feed line for the epichlorohydrin, which is arranged parallel to the stirrer shaft and opens into the reactor chamber with a plurality of line ends or line openings at different levels,
at least one evaporator unit, preferably a film evaporator and/or short-path evaporator,
at least one reaction vessel for performing the hydrolysis,
at least one reaction vessel for performing the subsequent hydrolysis, and
at least one additional short-path evaporator,
characterised in that the reaction chamber in which the reaction of epichlorohydrin with glycerol takes place is subdivided by constrictions in cross-section into at least two, preferably more than three, reaction zones or chambers, that the feed line for the epichlorohydrin is located within the reactor chamber at a distance from the outer periphery of the stirrer mechanism or mechanisms and that at least one line end or one line opening of the feed line for the epichlorohydrin opens into each reaction zone or chamber and at least one stirrer mechanism is fastened to the stirrer shaft.

11. An apparatus according to Claim 10, characterised in that the constrictions in cross-section consist of partition plates, baffle plates or separating discs, which have a plurality of holes or passage openings for the reaction mixture.

12. An apparatus according to Claims 10 or 11, characterised in that the partition plates, baffle plates or separating discs are circular and are arranged horizontally at various levels in the reactor chamber, with the partition plates, baffle plates or separating discs each having a central opening for the stirrer shaft to pass through and an opening for the feed line for the epichlorohydrin to pass through.

13. An apparatus according to one or more of Claims 10 to 12, characterised in that a gap which is not sealed is formed between the edge of the partition plates, baffle plates or separating discs and the reactor wall.

14. An apparatus according to one or more of Claims 10 to 13, characterised in that the partition plates, baffle plates or separating discs are at approximately the same distance from each other.

15. An apparatus according to one or more of Claims 10 to 14, characterised in that the line ends or openings of the feed line for the epichlorohydrin are designed as venturi nozzles.

## Revendications

1. Procédé de préparation de diglycérol à faible teneur en constituants cycliques par réaction de glycérol avec de l'épichlorhydrine à des températures de
20 à 120°C, de préférence de
50 à 100°C,
en présence d'un catalyseur acide et dans un rapport molaire du glycérol à l'épichlorhydrine de 20:1 à 1:1, de préférence de 12:1 à 5:1, procédé dans lequel on élimine ensuite du mélange réactionnel obtenu le glycérol en excès et on fait réagir le mélange chlorhydrine-éther ou mélange réactionnel restant, à une température de
60 à 120°C, de préférence de
80 à 110°C,
(correspondant à la teneur du mélange réactionnel en chlore à liaison organique), avec un milieu à réaction alcaline, de préférence une solution aqueuse à réaction alcaline, caractérisé en ce que, pour préparer le diglycérol en continu, on introduit l'épichlorhydrine nécessaire à la réaction en plusieurs points, ou zones réactionnelles, séparés les uns des autres dans la direction d'écoulement, d'au moins un réacteur continu, en ce que le mélange réactionnel qui a réagi est largement débarrassé ou séparé du glycérol en excès dans au moins une installation d'évaporation et le mélange chlorhydrine-éther restant est, au moins dans une première étape, hydrolysé par une solution d'un carbonate alcalin dans un excès de
5 à 40 moles %, de préférence de
15 à 25 moles %,
calculé sur base du chlore à liaison organique du chlorhydrine-éther, et dans une plage de pH de
6,5 à 9,5, de préférence
7 à 8,5,
et l'hydrolyse est terminée, dans une deuxième étape, au moyen d'un hydroxyde alcalin, de préférence une solution aqueuse d'hydroxyde alcalin, dans une plage de pH supérieure à 10; en ce que, à la fin ou largement vers la fin de l'hydrolyse, on ajuste la solution de produit brut à une valeur du pH de
5,5 à 8, de préférence de
6 à 7,
par addition d'un acide minéral, de préférence l'acide chlorhydrique et on évapore la solution obtenue, les sels émergents étant séparés et la solution de diglycérol ayant une teneur résiduelle en sels minéraux inférieure à 8 % en poids étant ensuite débarrassée du diglycérol cyclique et de fractions résiduelles de glycérol par distillation dans un évaporateur flash.

2. Procédé selon la revendication 1, caractérisé en ce que l'épichlorhydrine est introduite sous la forme de deux courants partiels ou plus dans un réacteur continu subdivisé en deux chambres individuelles, ou zones réactionnelles, ou davantage par des rétrécissements transversaux, au moins un courant partiel étant introduit de préférence dans chaque chambre individuelle ou zone réactionnelle.

3. Procédé selon la revendication 1 ou 2, caractérisé en ce que le courant total d'épichlohydrine nécessaire à la réaction est subdivisé en deux courants partiels et un premier courant partiel est introduit en plusieurs points, ou zones réactionnelles, séparés spatialement les uns des autres dans la direction de l'écoulement, d'un premier réacteur continu et, ensuite, le mélange réactionnel est envoyé en continu dans un deuxième réacteur continu, où le deuxième courant partiel d'épichlorhydrine est introduit de préférence en plusieurs points, ou zones réactionnelles, séparés spatialement les uns des autres dans la direction de l'écoulement.

4. Procédé selon une ou plusieurs des revendications 1 à 3, caractérisé en ce que la teneur (du mélange réactionnel après réaction du glycérol avec l'épichlorhydrine) en glycérol utilisé en excès est réduite en dessous de 20 % en poids (par rapport au mélange réactionnel), de préférence en dessous de 10 % en poids dans au moins une installation d'évaporation opérant en continu.

5. Procédé selon la revendication 4, caractérisé en ce que l'on utilise comme installation d'évaporation un évaporateur à couche mince et/ou un évaporateur flash, de préférence une combinaison des deux types d'évaporateur, et on recycle dans le procédé le glycérol obtenu comme produit de tête.

6. Procédé selon une ou plusieurs des revendications 1 à 5, caractérisé en ce qu'on utilise comme solution de carbonate alcalin une solution de soude de
plus de 1,5 molaire, de préférence de
plus de 2 molaire.

7. Procédé selon une ou plusieurs des revendications 1 à 6, caractérisé en ce qu'on utilise comme solution aqueuse d'hydroxyde alcalin une lessive sodique à plus de 10 % en poids, de préférence une lessive sodique à plus de 35 % en poids.

8. Procédé selon une ou plusieurs des revendications 1 à 7, caractérisé en ce que le temps de séjour du mélange réactionnel dans la première étape de l'hydrolyse est ajusté à une durée supérieure à 1 heure, de préférence supérieure à 1,5 heure, et la durée de séjour dans la deuxième étape d'hydrolyse est ajusté à plus de 0,5 heure, de préférence à plus de 0,75 heure.

9. Procédé selon une ou plusieurs des revendications 1 à 8, caractérisé en ce que la solution de produit brut traitée par un acide minéral est évaporée dans un évaporateur flash et est ensuite débarrassée des sels émergents par filtration ou centrifugation.

10. Dispositif permettant de réaliser le procédé selon les revendications 1 à 9, comprenant :
un réacteur ou une cuve de réaction, de préférence un réacteur continu agencé verticalement, comportant un corps de réacteur cylindrique, avec au moins un orifice d'admission pour le glycérol et le catalyseur à l'extrémité inférieure et au moins un orifice d'évacuation du mélange réactionnel à l'extrémité supérieure; au moins un arbre d'agitation agencé en position coaxiale et centrale dans le réacteur ou la cuve de réaction et sur lequel est fixé un dispositif d'agitation; et une conduite d'alimentation pour l'épichlorhydrine, qui est agencée parallèlement à l'arbre d'agitation et qui débouche dans l'espace de réaction à divers niveaux en hauteur par plusieurs extrémités ou ouvertures de la conduite,
au moins une installation d'évaporation, de préférence un évaporateur à couche mince et/ou un évaporateur flash,
au moins une cuve de réaction pour effectuer l'hydrolyse,
au moins une cuve de réaction pour effectuer la post-hydrolyse,
au moins un autre évaporateur flash, caractérisé en ce que l'espace réactionnel, dans lequel a lieu la réaction de l'épichlorhydrine et du glycérol, est subdivisé en au moins deux, de préférence plus de trois zones ou chambres réactionnelles par des rétrécissements transversaux, en ce que la conduite d'alimentation pour l'épichlorhydrine est agencée à l'intérieur de l'espace réactionnel, à distance de la périphérie externe du ou des dispositifs d'agitation, et en ce qu'au moins une extrémité ou une ouverture de la conduite d'alimentation pour l'épichlorhydrine débouche dans chaque zone ou chambre réactionnelle et au moins un dispositif d'agitation est fixé à l'arbre d'agitation.

11. Dispositif selon la revendication 10, caractérisé en ce que les rétrécissements transversaux sont constitués de plaques de fond intermédiaires, de chicanes ou de disques séparateurs qui présentent plusieurs perforations ou ouvertures de passage pour le mélange réactionnel.

12. Dispositif selon la revendication 10 ou 11, caractérisé en ce que les plaques de fond intermédiaires, les chicanes ou les disques séparateurs ont une forme circulaire et sont disposés horizontalement sur plusieurs niveaux en hauteur dans l'espace réactionnel, les plaques de fond intermédiaires, les chicanes ou les disques séparateurs présentant respectivement une ouverture centrale pour permettre le passage de l'arbre d'agitation et une ouverture permettant le passage de la conduite d'alimentation pour l'épichlorhydrine.

13. Dispositif selon une ou plusieurs des revendications 10 à 12, caractérisé en ce qu'un intervalle non étanché est ménagé entre le bord des plaques de fond intermédiaires, des chicanes ou des disques séparateurs et la paroi du réacteur.

14. Dispositif selon une ou plusieurs des revendications 10 à 13, caractérisé en ce que les plaques de fond intermédiaires, les chicanes ou les disques séparateurs sont séparés les uns des autres approximativement de la même distance.

15. Dispositif selon une ou plusieurs des revendications 10 à 14, caractérisé en ce que les extrémités ou les ouvertures de la conduite d'alimentation pour l'épichlorhydrine se présentent sous la forme de tubes de Venturi.
